# EUROPÄISCHE PATENTSCHRIFT

(11) **EP 4 038 225 B1**
(45) Veröffentlichungstag und Bekanntmachung des Hinweises auf die Patenterteilung: **25.10.2023**
(21) Anmeldenummer: 21726609.7
(22) Anmeldetag: 11.05.2021
(51) Int. Cl.: D03D 15/63, D03D 41/00, D03C 13/00, D03C 9/06

(54) **WEBSTUHL MIT BEWEGLICHEN FÜHRUNGSBÄUMEN**
LOOM WITH MOVABLE GUIDE BEAMS
MÉTIER À TISSER AVEC ENSOUPLES DE GUIDAGE MOBILES

(30) Priorität: 12.05.2020 DE 202020002061 U
(43) Veröffentlichungstag der Anmeldung: 10.08.2022
(73) Patentinhaber: Castens, Sybille, 26197 Großenkneten (DE)
(72) Erfinder: Castens, Sybille, 26197 Großenkneten (DE)
(74) Vertreter: Heiland, Karsten
(86) Internationale Anmeldenummer: PCT/EP2021/062496
(87) Internationale Veröffentlichungsnummer: WO 2021/228864

(56) Entgegenhaltungen:
- DE-C- 361 359
- DE-U- 1 733 292
- FR-A- 554 820
- IT-A1- FI20 130 079
- JP-A- 2005 015 954
- US-A1- 2016 076 174

## Beschreibung

Der hier beschriebenen Erfindung liegt folgendes Problem zugrunde: In Flach-Webstühlen wird ein Fach nach vier Punkten begrenzt und ist damit umschlossen
nach oben: von Kettfäden im nach oben gehaltenen Zustand,
nach unten: von Kettfäden im nach unten gehaltenen Zustand,
nach vorn: von bisher Gewebtem auf dem Warenbaum und
nach hinten: von einem Webblatt.

Dadurch ist es bislang unabdingbar, einen Schussfaden von der einen zur anderen Seite des Webstuhles durch das jeweilige Fach hindurch zu transportieren. Dazu dienen nach aktuellem Stand der Technik: - Schützen, auf denen ein Vorrat an Schussfaden aufgespult ist, - Greifer, die den Schussfaden durch das Fach reichen, der nach jedem Eintrag abgeschnitten wird, - Düsen, die den Schussfaden mit Luft- oder Wasserkraft transportieren, und der nach jedem Eintrag abgeschnitten wird.

Daher ist der Durchmesser des Schussfadens durch den Durchmesser des Fachs und auch seine Festigkeit durch die jeweils verwendeten Techniken begrenzt. Die Länge des Schussfadens wird durch die Aufnahmekapazität von Spule und Greifer, und bei Düsen annähernd durch die Breite der Ware begrenzt.

Die FR 554 820 A offenbart einen mechanischen Webstuhl für die Herstellung von Stoffen aus Holzwolle. Kettfäden werden entlang schwenkbarer Hebel oder Bögen zugeführt, wobei der Warenbaum unterhalb der Hebel oder Bögen gehalten ist und Schussfäden durch eine auf einer verstellbaren geneigten Bahn laufende oder mittels Schlitten verschiebbaren Spule zugeführt werden.

Die DE 361 359 C zeigt einen Webstuhl zum Verweben von Holzwollseilen. Kettseile werden in Abwärtsrichtung zugeführt. Ein Webfach ist durch die Kettseile haltende Fachbildungsbügel veränderbar.

Die JP 2005 015954 A zeigt einen Flachwebstuhl, mit dem das Einbringen eines zusätzlichen Musterfadens möglich ist.

Die IT FI20 130 079 A1 zeigt ein Gewebe mit Kettfäden aus Leder und aus sonstigem Garn, während die Schussfäden aus sonstigem Garn bestehen.

So ist es nicht möglich, ein endloses Material durch das Fach zu bringen. Loses oder lose verbundenes Material kann mit den herkömmlichen Techniken überhaupt nicht an seinen Platz zwischen den unteren und oberen Fäden gebracht werden.

Dieses Problem wird mit den im Anspruch 1 aufgeführten Merkmalen gelöst, vorzugsweise auch mit den Merkmalen der weiteren Ansprüche.

Das geschieht dadurch, dass der Webstuhl im Vergleich zu herkömmlichen Flach-Webstühlen um 90° gedreht wurde, so dass der Warenbaum unten liegt. Weiter wurde das starre Litzen-System aufgegeben, und statt der Anordnung der Litzen in Schäften wurde jeder einzelne Faden durch einen ihm zugeordneten Haken geführt, der reihenweise in einem Führungsbaum verankert ist. Zwischen diesen Hakenreihen von vorderem und hinterem Führungsbaum hindurch kann nun von oberhalb des Webstuhls sogar ein loses Material auf den Warenbaum gelegt werden, wobei die Kettfäden als vordere und hintere Begrenzung dienen - nur deren Abstand zueinander begrenzen die Auswahl des losen Materials. Der Warenbaum befindet sich unterhalb des jeweils gebildeten Fachs und bildet durch Schwerkraft eine natürliche Sperre nach unten hin.

Vorteilhafterweise stehen sich die Haken auf den zwei gegenüberliegenden Führungsbäumen versetzt gegenüber, mit einem Versatz in Längsrichtung der Führungsbäume.

Außerdem soll von den zwei Führungsbäumen mindestens einer beweglich sein, um mit einem Nähern der Führungsbäume zueinander die Haken ineinander greifen zu lassen, nämlich die Haken des einen Führungsbaums zwischen die Haken des anderen Führungsbaums treten zu lassen.

Mit der Erfindung wird erreicht, dass zwei unterschiedliche Zustände im Wechsel zwei unterschiedliche Fächer bilden. Im offenen Zustand (V-Position) kann jedes Material eingebracht werden, das
- von den Kettfäden und dem Warenbaum getragen werden kann,
- von den Kettfäden nach vorn und nach hinten hin in seiner Position gehalten werden kann,
- im Durchmesser die Schenkellänge der Kettfäden und den Abstand zum Warenbaum als Winkelhalbierende je Eintragsvorgang nicht überschreitet.

Der Fachwechsel findet statt, indem der vordere Führungsbaum dem hinteren Führungsbaum angenähert wird, bis sich durch die nun abwechselnd verschränkten vorderen und hinteren Haken ein Fach bildet, das nach oben hin geschlossen ist (verschränkte Position). Im nach oben geschlossenen Zustand wird dann ein Faden auf herkömmliche Art eingebracht, der das im offenen Zustand eingebrachte Material nach oben hin stabilisiert.

Als nächster Schritt wird zum Ausgangszustand zurückgekehrt und das Weben beginnt von vorn.

Auf diese Weise können Kabel, Schläuche und andere Materialien verwebt werden, die keinesfalls durchtrennt werden dürfen, und die länger sind, als ein Schütze auf seiner Spule bevorraten kann. Auch loses Material kann eingebracht werden, das bislang nicht auf Schützen bevorratet werden konnte, oder nicht mittels Greifer durchgereicht, oder auch nicht durch einen Luft- oder Wasserstrahl durchs Fach getrieben werden konnte.

Optional und erfindungsgemäß können Warenbaum und Führungsbäume derart angeordnet und ausgerichtet sein, dass die Kettfäden des einen Führungsbaums eine erste Kettfäden-Ebene und die Kettfäden des anderen Führungsbaums eine zweite Kettfäden-Ebene bilden, und dass die Kettfäden-Ebenen durch Relativbewegung der Führungsbäume mit Haken relativ zueinander bewegbar sind. Insbesondere erstrecken sich die Kettfäden-Ebenen von den Führungsbäumen bis zum fertiggestellten Gewebe oberhalb des Warenbaums. Durch zusätzlich vorgesehene Führungsorgane können die Kettfäden-Ebenen auch gekrümmt oder abgewinkelt sein, insbesondere oberhalb der Führungsbäume und in Richtung auf die Kettbäume.

Optional und erfindungsgemäß können die Kettfäden-Ebenen in einer Relativposition der Führungsbäume eine offene V-Position bilden, mit einem nach oben offenen Fach und mit dem Warenbaum unterhalb des Fachs.

Optional und erfindungsgemäß können die Kettfäden-Ebenen mittels der Führungsbäume aus der offenen V-Position in eine verschränkte Position und wieder zurück bewegbar sein. Die Veränderung der Position der Kettfäden-Ebenen bewirkt den Übergang von der V-Position in die verschränkte Position und wieder zurück.

Optional und erfindungsgemäß können die Kettfäden-Ebenen zumindest in der verschränkten Position abgewinkelt oder gekrümmt sein. Die Abwinkelung ist insbesondere durch die Führung der Kettfäden an den Haken gegeben. Eine Abwinkelung oder Krümmung der Kettfäden-Ebenen kann aber auch in der V-Position gegeben sein, je nach Anordnung der Kettbäume und möglicher weiterer Führungsorgane für die Kettfäden.

Optional und erfindungsgemäß können die Kettfäden-Ebenen zwischen der V-Position und der verschränkten Position eine bündige, neutrale Position einnehmen. Dabei kommen die Kettfäden-Ebenen zur Deckung.

Optional und erfindungsgemäß können wenigstens zwei Kettbäume vorgesehen sein, jeder zur Aufnahme der Kettfäden einer der Kettfäden-Ebenen, wobei die Kettbäume mit Abstand zueinander angeordnet sind und der Abstand vorzugsweise zumindest einer größten Weite des V-Fachs entspricht. Der Ort der größten Weite ist die höchste Stelle, an der die Kettfäden zum Führungsbaum schräg abwärts verlaufen, also nicht horizontal oder aufwärts geführt sind.

Optional und erfindungsgemäß können zwei Kettbäume mit Abstand zueinander angeordnet sein, wobei jeder der Kettbäume einem der Führungsbäume zugeordnet ist und die Kettfäden hält, die vom zugeordneten Führungsbaum geführt werden. Erster Führungsbaum mit erstem Kettbaum stehen einem zweiten Führungsbaum mit zweitem Kettbaum gegenüber.

Erfindungsgemäß können die Kettfäden durch die Anordnung der Kettbäume und Anordnung und Bewegbarkeit der Führungsbäume relativ zueinander zwischen einer oben offenen V-Position und einer verschränkten Position bewegbar sein. In verschränkter Position bilden die Kettfäden des einen Führungsbaums mit den Kettfäden des anderen Führungsbaums ein geschlossenes Fach für einen Schussfaden, der auf herkömmliche Art und Weise in das Fach einzubringen ist.

Optional und erfindungsgemäß können die Haken in der verschränkten Position derart miteinander verzahnt sein, dass die Haken des einen Führungsbaums zwischen die Haken des anderen Führungsbaums und an diesen vorbei bewegt sind. Auf diese Weise kommen die Haken des einen Führungsbaums bis in eine Position dicht an den anderen Führungsbaum.

Optional und erfinderisch können die Führungsbäume unterteilt sein in axial (in Längsrichtung) aufeinanderfolgende Führungsbaumsegmente, wobei die Führungsbaumsegmente unabhängig voneinander bewegbar sind. An jedem Führungsbaumsegment können ein oder mehrere Haken angeordnet sein. Insbesondere sind die Führungsbaumsegmente bewegbar zwischen der V-Position und der verschränkten Position. Alternativ oder zusätzlich können die Führungsbaumsegmente verdrehbar sein, vorzugsweise um eine Drehachse parallel zur Hakenlängsrichtung. Verdrehbare Führungsbaumsegmente sind vorzugsweise mit zwei oder mehr Haken versehen.

Optional und erfinderisch können die Haken offen, geschlossen oder geschlossen aber öffenbar ausgebildet sein. Geschlossene Haken entsprechen Ringen. Offene Haken weisen einen Spalt auf zum Eintritt der Kettfäden und öffenbare Haken weisen einen verschließbaren Spalt auf.

Optional und erfinderisch können die Haken eine endseitige Hakenkrümmung von mindestens 280 Grad aufweisen, vorzugsweise von mindestens 370 Grad. Dabei kann das Ausmaß der Hakenkrümmung auch vom Übergang zwischen der endseitigen Hakenkrümmung und dem übrigen Teil des Hakens abhängen. Vorteilhaft ist ein Ausmaß der Hakenkrümmung derart, dass der aufgenommene Kettfaden bei der Hin- und Herbewegung des Hakens innerhalb der Hakenkrümmung verbleibt.

Optional und erfindungsgemäß kann ein erster Rahmen mit Kettbaum und Führungsbaum relativ bewegbar sein zu einem zweiten Rahmen mit Kettbaum und Führungsbaum, wobei vorzugsweise nur einer der Rahmen bewegbar ist. Die Anordnung von Kettbaum und Führungsbaum in einem gemeinsamen Rahmen vereinfacht den Aufbau des Webstuhls, ebenso die fixe Anordnung des einen Rahmens, während nur der andere Rahmen bewegbar ist.

Gegenstand der Erfindung ist auch ein Verfahren zum Herstellen eines Gewebes, unter Verwendung eines erfindungsgemäßen Webstuhls, siehe Anspruch 17.

Erfindungsgemäß kann in einer verschränkten Position des Webstuhls ein Schussfaden ins geschlossene Fach eingebracht werden, wobei in einer V-Position des Webstuhls wenigstens ein Materialstrang in das dann offene Fach gelegt wird. Dabei unterscheidet sich jedoch der Materialstrang vom Schussfaden.

Ein Gewebe mit Kettfäden und Schussfäden, welches jedoch nicht Bestandteil der Erfindung ist, ist zumindest teilweise auf einem erfindungsgemäßen Webstuhl herstellbar.

Im Gewebe können parallel zu den Schussfäden und mit diesen abwechselnd Materialstränge oder wenigstens ein durchgehender Materialstrang vorgesehen sein. Das Gewebe kann abwechselnd zu den Schussfäden auch mehrere und/oder unterschiedliche Materialstränge aufweisen.

Als Materialstränge oder als Materialstrang können
- ein Wollstrang, oder
- ein Bewässerungsschlauch, oder
- Kombinationen daraus
vorgesehen sein. Der Wollstrang ist dabei aus einem oder mehreren Kammzügen Wolle gebildet.

Durch die Herstellung auf dem erfindungsgemäßen Webstuhl kann das nicht erfindungsgemässe Gewebe völlig neue und unerwartete Materialkombinationen aufweisen. Ein vorteilhaftes Gewebe weist als Materialstränge eine Kombination aus einem Kammzug Wolle mit einem Bewässerungsschlauch auf. Das Gewebe kann als Abdeckung für zu bewässerndes Erdreich dienen. Pflanzen können durch die Wolle hindurch wachsen. Die Wolle dient als Dünger oder kann zusätzlich mit Düngermaterial versehen sein. Außerdem verhindert die Wolle ein schnelles Verdunsten des Wassers. Unbehandelte Wolle wirkt hydrophob, so dass das Wasser an der Wolle abläuft.

Weitere Merkmale der Erfindung ergeben sich aus der Beschreibung im Übrigen und aus den Ansprüchen.

Anhand der Abbildungen von **Fig. 1-11** wird die Technik verdeutlicht. Es zeigen:
- **Fig. 1** und **Fig. 2**: eine einfache Seitenansicht,
- **Fig. 3** und **Fig. 4**: diese Seitenansichten zusätzlich perspektivisch,
- **Fig. 5**: eine seitliche Draufsicht auf einen Rahmen,
- **Fig. 6**: einen Webstuhl mit zwei Rahmen in Seitenansicht in V-Position,
- **Fig. 7**: den Webstuhl gemäß **Fig. 6** in veränderter Position,
- **Fig. 8**: Führungsbäume mit Haken in verschränkter Position,
- **Fig. 9**: Führungsbäume mit Haken in offener V-Position,
- **Fig. 10**: Führungsbäume mit Haken in neutraler Position,
- **Fig. 11**: einen einteiligen Führungsbaum und einen segmentierten Führungsbaum,
- **Fig. 12**: ein Gewebe im Webstuhl
- **Fig. 13**: einen Schnitt durch die Fäden bzw. das Material in **Fig. 12****.**

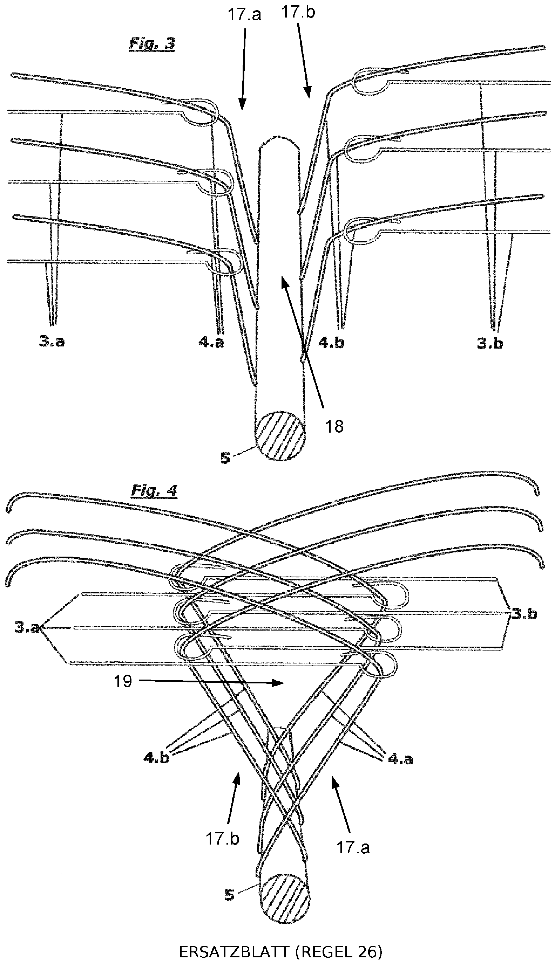

Zur Übersichtlichkeit wurden nur jene Bauteile dargestellt, die für die Erfindung wesentlich sind. Die Anzahl der Haken wurde in **Fig. 3** und **Fig. 4** auf drei Haken je Führungsbaum beschränkt, um das Prinzip der Technik zu erläutern.

Für alle Abbildungen gilt: Die vorderen Kettfäden 4.a verlaufen vom vorderen Kettbaum 1.a durch die im vorderen Führungsbaum 2.a verankerten vorderen Haken 3.a zum Warenbaum 5. Die hinteren Kettfäden 4.a verlaufen vom hinteren Kettbaum 1.b durch die im hinteren Führungsbaum 2.b verankerten hinteren Haken 3.b zum Warenbaum 5.

Die Unterteilung in vorderen und hinteren Führungsbaum ist willkürlich, dient nur der Unterscheidung und kann auch umgekehrt sein oder durch rechten und linken oder ersten und zweiten Führungsbaum ersetzt werden. Gleiches gilt für andere paarweise vorhandene Bauteile.

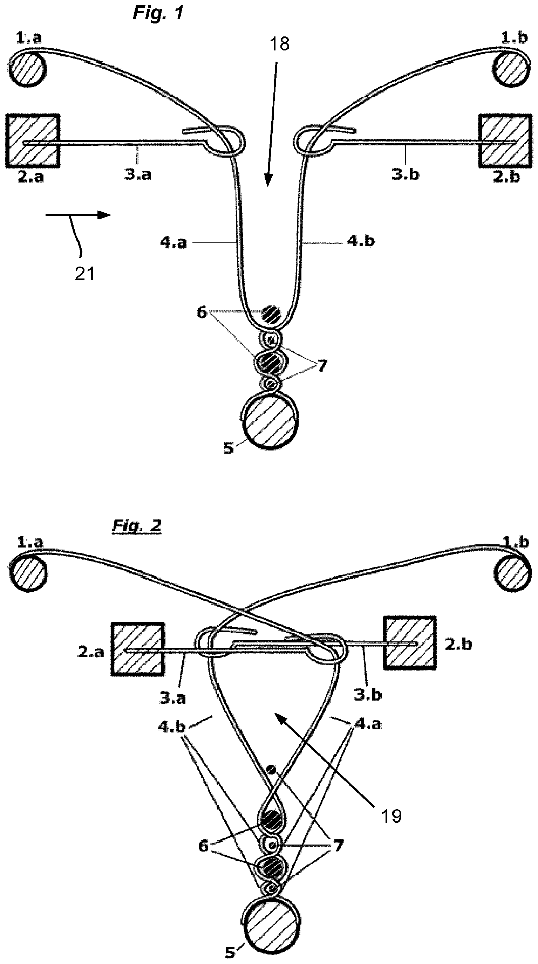

**Fig. 1** und **Fig. 3** stellen den offenen Fach-Zustand dar, nämlich ein offenes Fach 18, während dessen zwischen den sich nicht verschränkenden Haken 3.a und 3.b der Eintrag von endlosem, losem oder lose verbundenem Material 6 erfolgt.

**Fig. 2** und **Fig. 4** zeigen den geschlossenen Zustand des Fachs, nämlich ein geschlossenes Fach 19, in dem unter den sich verschränkenden Haken 3.a und 3.b ein herkömmlicher Schusseintrag 7 stattfindet.

Wie in den **Fig. 3 und 4** gut ersichtlich, bilden die vorderen Kettfäden 4.a eine gemeinsame Kettfäden-Ebene 17.a und die hinteren Kettfäden 4.b eine hintere Kettfäden-Ebene 17.b. In **Fig. 3** bilden die Kettfäden-Ebenen 17.a, 17.b das offene V-Fach. In **Fig. 4** sind die Kettfäden-Ebenen 17.a, 17.b gegeneinander verschränkt und bilden in Verbindung mit der Umlenkung der Kettfäden 4.a, 4.b im Bereich der Haken 3.a, 3.b ein geschlossenes Fach.

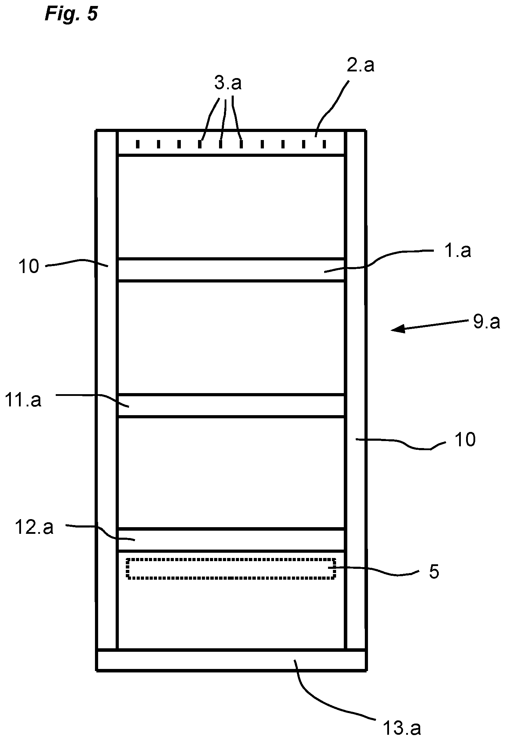

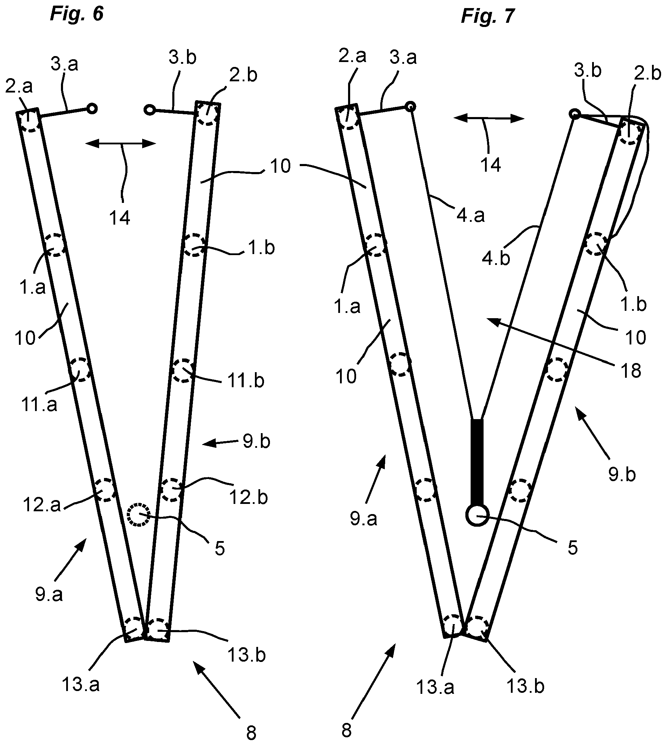

Ein erfindungsgemäßer Webstuhl 8 kann im Wesentlichen zwei Rahmen 9.a, 9.b aufweisen, wie in den **Fig. 6 und 7** dargestellt. **Fig. 5** zeigt den Rahmen 9.a in einer Frontansicht. Als Bestandteile des Rahmens 9.a sind erkennbar: äußere aufrechte Längsholme 10, der vordere Führungsbaum 2.a als oberer Abschluss, mit Abstand darunter der vordere Kettbaum 1.a, darunter mit Abstand zwei Stützbäume 11.a, 12.a und als unteren Abschluss ein Gelenkbaum 13.a.

Die beiden Rahmen 9.a, 9.b sind im Bereich der Gelenkbäume 13.a, 13.b in nicht näher dargestellter Weise gelenkig miteinander verbunden, so dass die Rahmen 9.a, 9.b aufeinander zu und in Gegenrichtung schwenkbar sind, siehe Doppelpfeil 14.

Vorzugsweise ist einer der beiden Rahmen 9.a, 9.b feststehend gelagert, während der andere Rahmen schwenkbar gehalten ist. Es können aber auch beide Rahmen 9.a, 9.b schwenkbar angeordnet sein.

**Fig. 7** zeigt die offene V-Position des Webstuhls 8 bzw. der Kettfäden 4.a, 4.b. In **Fig. 6** sind die beiden Rahmen 9.a, 9.b etwas enger zusammengeschwenkt als in **Fig. 7****.** Es handelt sich lediglich um eine Zwischenposition auf dem Weg in die verschränkte Position, welche sich aus den **Fig. 2** **und** **4** ergibt.

Der Kettfaden 4.b ist in **Fig. 7** oberhalb des Hakens 3.b um den Führungsbaum 2.b herum und nach unten zum Kettbaum 1.b geführt und wird dort gehalten. Analog gilt dies für den Kettfaden 4.a, dort aber nicht explizit eingezeichnet.

In der Darstellung der **Fig. 7** verläuft der Kettfaden 4.b vom Führungsbaum 2.b zum Kettbaum 1.b lose. Diese Darstellung wurde nur gewählt, um den Kettfaden 4.b an dieser Stelle deutlich zeigen zu können. Tatsächlich ist der Kettfaden bis zum Kettbaum 1.b vorzugsweise stramm gespannt.

In **Fig. 6** ist der Warenbaum 5 nur gestrichelt angedeutet und die Kettfäden 4.a, 4.b sind aus Gründen der besseren Übersichtlichkeit nicht eingezeichnet.

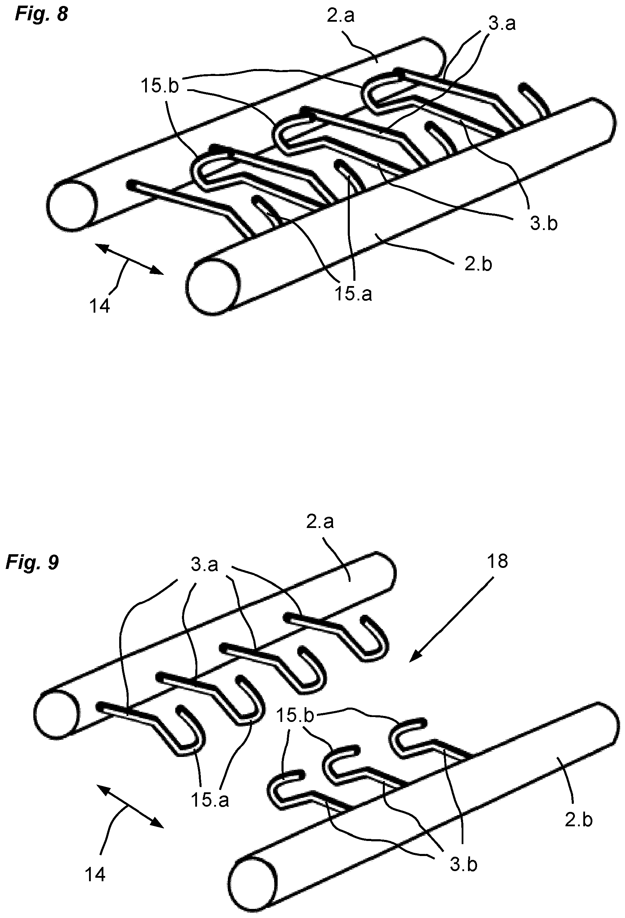

Die **Fig. 8, 9****,** **10** zeigen die mögliche Relativbewegung der Führungsbäume 2.a, 2.b mit den Haken 3.a, 3.b. **Fig. 9** entspricht in etwa der Darstellung in **Fig. 7** mit der offenen V-Position und weit auseinander bewegten Führungsbäumen 2.a, 2.b, auch passend zu den **Fig. 1** **und** **3****.** Endseitige Krümmungen 15 zur Aufnahme der Kettfäden 4.a, 4.b der Haken 3.a, 3.b weisen in Richtung des Pfeils 14 einen deutlichen Abstand voneinander auf. Die Haken 3.a, 3.b liegen vorzugsweise etwa in einer gemeinsamen horizontalen Ebene.

**Fig. 8** zeigt die verschränkte Position, analog den **Fig. 2****,** **4****.** Die Führungsbäume 2.a, 2.b sind gegeneinander bewegt, soweit dies gegen die Erstreckung der Haken 3.a, 3.b möglich ist. Die Haken 3.a des Führungsbaums 2.a sind mit den Haken 3.b des Führungsbaums 2.b verzahnt, so dass die endseitigen Krümmungen 15.a der Haken 3.a an den endseitigen Krümmungen 15.b der Haken 3.b vorbei bewegt sind in Richtung auf den gegenüberliegenden Führungsbaum 2.b bzw. 2.a.

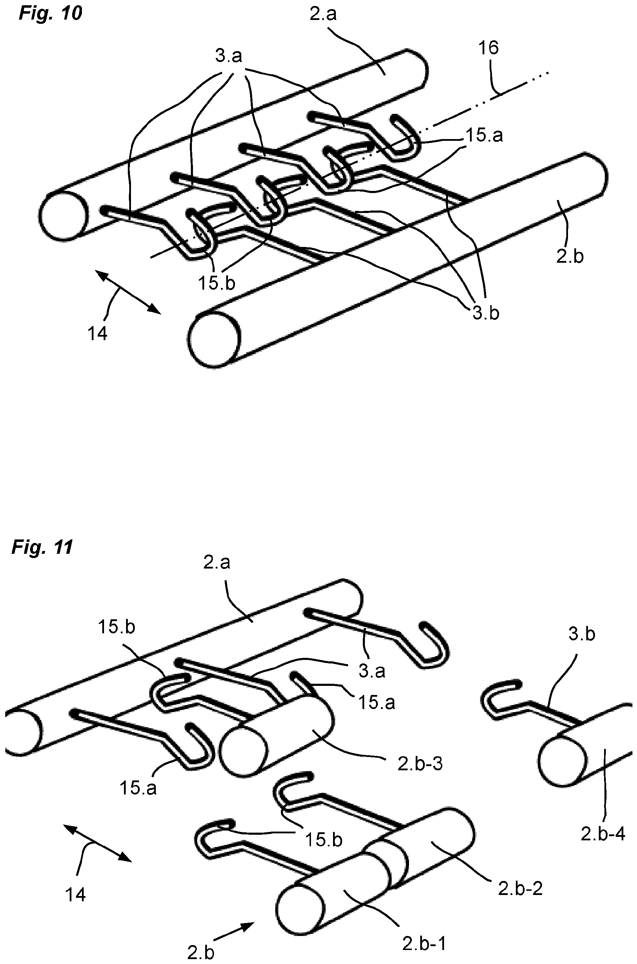

**Fig. 10** zeigt eine Zwischenstellung der Führungsbäume 2.a, 2.b, in der die endseitigen Krümmungen 15.a, 15.b beider Führungsbäume 2.a, 2.b entlang einer gemeinsamen Linie 16 ausgerichtet sind. Die Zwischenstellung kann auch als neutrale Position oder I-Position bezeichnet werden. In der praktischen Anwendung des Webstuhls wird die Zwischenposition in Normalfall ohne Stillstand passiert.

**Fig. 11** zeigt eine Besonderheit in der Gestaltung des Führungsbaums 2.b. Dieser ist in Längsrichtung unterteilt in einzelne Führungsbaumsegmente 2.b-1, 2.b-2, 2.b-3, 2.b-4 usw. Jedes Führungsbaumsegment ist unabhängig von den anderen Führungsbaumsegmenten in Richtung des Pfeils 14 bewegbar. Hierfür geeignete Halter und Antriebe sind nicht eingezeichnet. Beispielhaft ist das Führungsbaumsegment 2.b-3 in der verschränkten Position gemäß **Fig. 8** dargestellt, während die übrigen Führungsbaumsegmente die offene V-Position gemäß **Fig. 9** einnehmen. Durch die beschriebene Segmentierung des Führungsbaums 2.b ist die Erzeugung spezieller Webmuster möglich.

Durch die unabhängige Bewegbarkeit der Führungsbaumsegmente kann die Gleichförmigkeit der Kettfäden-Ebene 17.b aufgehoben werden. In der Position gemäß **Fig. 11** wäre ein Kettfaden am Führungsbaumsegment 2.b-3 nicht mehr Bestandteil der Kettfäden-Ebene 17.b.

In **Fig. 11** weist jedes Führungsbaumsegment 2.b-1 bis 2.b-4 jeweils einen Haken 3.b auf. Möglich ist auch eine andere Aufteilung, etwa mit zwei oder mehr Haken je Führungsbaumsegment.

Ein Verdrehen benachbarter Kettfäden ist beispielsweise möglich durch verdrehbare Führungsbaumsegmente mit je zwei Haken 3.b. Die Drehung erfolgt vorzugsweise um eine Drehachse parallel zum Pfeil 14.

In **Fig. 11** ist nur der Führungsbaum 2.b mit Führungsbaumsegmenten dargestellt. Alternativ oder zusätzlich kann auch der andere Führungsbaum 2.a segmentiert ausgebildet sein.

Die endseitigen Krümmungen 15.a, 15.b der Haken 3.a, 3.b sind in den **Figuren** vereinfacht dargestellt. Tatsächlich sind die Krümmungen 15.a, 15.b derart ausgebildet, dass mit ihnen die Kettfäden 4.a, 4.b in beide Richtungen des Pfeils 14 aktiv bewegt werden können. Um dies zu erreichen, können die endseitigen Krümmungen 15.a, 15.b auch als geschlossener Ring ausgebildet sein, oder als offener Ring, so dass die Kettfäden 4.a, 4.b durch Seitwärtsbewegung in die Krümmungen 15.a, 15.b eingelegt werden können. Zumindest sollten sich die endseitigen Krümmungen 15.a, 15.b über einen Winkel von mehr als 270 Grad erstrecken, vorzugsweise mehr als 360 Grad, je nach Gestaltung des Übergangs zwischen den endseitigen Krümmungen und dem übrigen Teil des Hakens 3.a, 3.b.

Mit dem erfindungsgemäßen Webstuhl 8 können Produkte ganz eigener Art hergestellt werden. In das offene Fach 18 der V-Position gemäß den **Fig. 1****,** **3****,** **7** **und** **9** können nicht-schussfähige Materialien eingelegt werden. Nicht-schussfähige Materialien sind beispielsweise besonders dicke, flauschige, steife und/oder endlose Materialien. Auch Kombinationen dieser Materialien sind möglich. Beispielsweise kann eine Erdreichabdeckung mit Bewässerungsfunktion hergestellt werden, indem in der offenen V-Position jeweils ein Wollstrang 6-1 zusammen mit einem endlosen Bewässerungsschlauch 6-2 eingelegt wird.

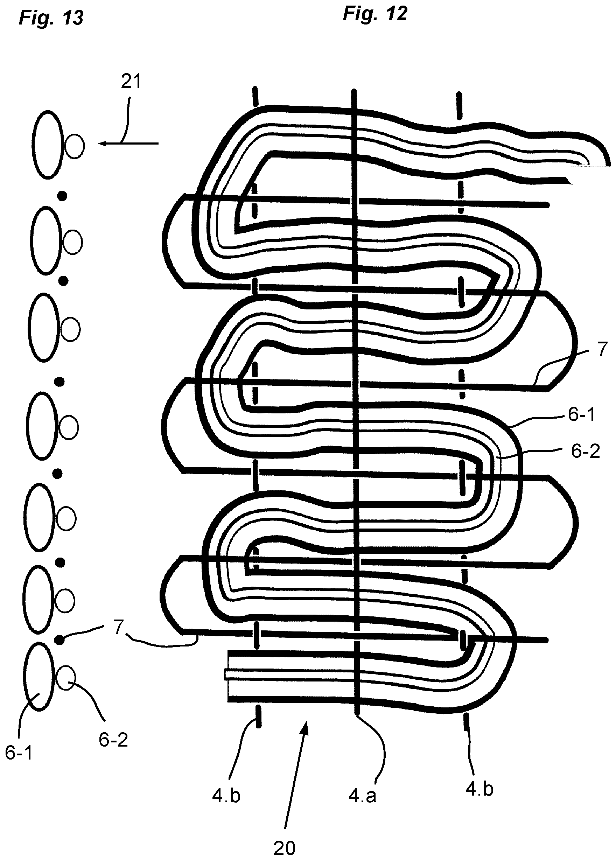

Ein derart gebildetes Gewebe 20 während des Webens zeigt **Fig. 12****,** in einer Blickrichtung gemäß Pfeil 21 in **Fig. 1** **und** **13****.** In **Fig. 13** sind die in Schussrichtung eingelegten Fäden 7 und Materialien 6-1, 6-2 im Schnitt gezeigt, korrespondierend zu **Fig. 12****.** Der Schussfaden 7, welcher auch als Stabilisierungsfaden bezeichnet werden kann, wechselt mit der Kombination aus Wollstrang 6-1 und Bewässerungsschlauch 6-2 ab. Dabei liegt der Bewässerungsschlauch 6-2 neben bzw. vor dem Wollstrang 6-1, kann aber auch vom Wollstrang eingeschlossen sein oder über/unter demselben liegen.

## Patentansprüche

1. Webstuhl zum Eintrag von endlosem, oder lose verbundenem oder stückweisem Material, mit Kettbäumen (1.a, 1.b) und Warenbaum (5), wobei die Kettfäden (4.a und 4.b) in Abwärtsrichtung von den Kettbäumen (1.a und 1.b) zum Warenbaum (5) verlaufen und dabei einzeln von Haken (3.a und 3.b) gehalten werden, die in Führungsbäumen (2.a und 2.b) zwischen oberen Kettbäumen (1.a und 1.b) und unterem Warenbaum (5) verankert sind, und wobei die Kettfäden (4.a, 4.b) durch die Anordnung der Kettbäume (1.a, 1.b) und Anordnung und Bewegbarkeit der Führungsbäume (2.a, 2.b) relativ zueinander zwischen einer oben offenen V-Position und einer verschränkten Position bewegbar sind.

2. Webstuhl nach Anspruch 1, **dadurch gekennzeichnet, dass** die Haken (3.a und 3.b) sich auf den zwei gegenüberliegenden Führungsbäumen (2.a und 2.b) versetzt gegenüber stehen.

3. Webstuhl nach Anspruch 1 oder 2, **dadurch gekennzeichnet, dass** von den zwei Führungsbäumen (2.a, 2.b) mindestens einer beweglich sein muss, um mit einem Nähern der Führungsbäume (2.a und 2.b) zueinander die Haken (3.a und 3.b) ineinandergreifen zu lassen.

4. Webstuhl nach einem der Ansprüche 1 bis 3, **dadurch gekennzeichnet, dass** Warenbaum (5) und Führungsbäume (2.a, 2.b) derart angeordnet und ausgerichtet sind, dass die Kettfäden (4.a) des einen Führungsbaums (2.a) eine erste Kettfädenebene (17.a) und die Kettfäden (4.b) des anderen Führungsbaums (2.b) eine zweite Kettfädenebene (17.b) bilden, und dass die Kettfädenebenen (17.a, 17.b) durch Relativbewegung der Führungsbäume (2.a, 2.b) mit Haken (3.a, 3.b) relativ zueinander bewegbar sind.

5. Webstuhl nach Anspruch 4, **dadurch gekennzeichnet, dass** die Kettfäden-Ebenen (17.a, 17.b) in einer Relativposition eine offene V-Position bilden, mit einem nach oben offenen Fach (18) und mit dem Warenbaum (5) unterhalb des Fachs (18).

6. Webstuhl nach Anspruch 5, **dadurch gekennzeichnet, dass** die Kettfäden-Ebenen (17.a, 17.b) mittels der Führungsbäume (2.a, 2.b) aus der offenen V-Position in eine verschränkte Position und wieder zurück bewegbar sind.

7. Webstuhl nach Anspruch 6, **dadurch gekennzeichnet, dass** die Kettfäden-Ebenen (17.a, 17.b) zumindest in der verschränkten Position abgewinkelt oder gekrümmt sein können.

8. Webstuhl nach Anspruch 6 oder 7, **dadurch gekennzeichnet, dass** die Kettfäden-Ebenen (17.a, 17.b) zwischen der V-Position und der verschränkten Position eine bündige Position einnehmen können.

9. Webstuhl nach Anspruch 1 oder einem der weiteren Ansprüche, **dadurch gekennzeichnet, dass** wenigstens zwei Kettbäume (1.a, 1.b) vorgesehen sind, jeder zur Aufnahme der Kettfäden (4.a, 4.b) einer der Kettfäden-Ebenen (17.a, 17.b), wobei die Kettbäume (1.a, 1.b) mit Abstand zueinander angeordnet sind.

10. Webstuhl nach Anspruch 5, **dadurch gekennzeichnet, dass** wenigstens zwei Kettbäume (1.a, 1.b) vorgesehen sind, jeder zur Aufnahme der Kettfäden (4.a, 4.b) einer der Kettfäden-Ebenen (17.a, 17.b), wobei die Kettbäume (1.a, 1.b) mit Abstand zueinander angeordnet sind und der Abstand zumindest einer größten Weite des Fachs (18) in der V-Position entspricht.

11. Webstuhl nach Anspruch 1 oder einem der weiteren Ansprüche, **dadurch gekennzeichnet, dass** zwei Kettbäume (1.a, 1.b) mit Abstand zueinander angeordnet sind, wobei jeder der Kettbäume (1.a, 1.b) einem der Führungsbäume (2.a, 2.b) zugeordnet ist und die Kettfäden (4.a, 4.b) hält, die vom zugeordneten Führungsbaum (2.a, 2.b) geführt werden.

12. Webstuhl nach Anspruch 11, **dadurch gekennzeichnet, dass** die Haken (3.a, 3.b) in der verschränkten Position derart miteinander verzahnt sind, dass die Haken (3.a) des einen Führungsbaums (2.a) zwischen die Haken (3.b) des anderen Führungsbaums (2.b) und an diesen vorbei bewegt sind.

13. Webstuhl nach Anspruch 1 oder einem der weiteren Ansprüche, **dadurch gekennzeichnet, dass** die Führungsbäume (2.a, 2.b) unterteilt sind in Längsrichtung aufeinander folgende Führungsbaumsegmente (2.b-1, 2.b-2, 2.b-3, 2.b-4), wobei die Führungsbaumsegmente (2.b-1, 2.b-2, 2.b-3, 2.b-4) unabhängig voneinander bewegbar sind.

14. Webstuhl nach Anspruch 1 oder einem der weiteren Ansprüche, **dadurch gekennzeichnet, dass** die Haken (3.a, 3.b) offen, geschlossen oder geschlossen aber öffenbar ausgebildet sind.

15. Webstuhl nach Anspruch 1 oder einem der weiteren Ansprüche, **dadurch gekennzeichnet, dass** die Haken (3.a, 3.b) eine endseitige Hakenkrümmung von mindestens 280 Grad aufweisen, vorzugsweise mindestens 370 Grad.

16. Webstuhl nach Anspruch 1 oder einem der weiteren Ansprüche, **dadurch gekennzeichnet, dass** ein erster Rahmen (9.a) mit Kettbaum (1.a) und Führungsbaum (2.a) relativ bewegbar ist zu einem zweiten Rahmen (9.b) mit Kettbaum (1.b) und Führungsbaum (2.b), wobei vorzugsweise nur einer der Rahmen bewegbar ist.

17. Verfahren zum Herstellen eines Gewebes, unter Verwendung eines Webstuhls (8) nach einem der voranstehenden Ansprüche, wobei in der verschränkten Position des Webstuhls (8) ein Schussfaden (7) in das geschlossene Fach (19) eingebracht wird und in der V-Position des Webstuhls (8) wenigstens ein Materialstrang (6) in das dann offene Fach (18) gelegt wird, und wobei sich der Materialstrang vom Schussfaden unterscheidet.

18. Verfahren nach Anspruch 17, **dadurch gekennzeichnet, dass** der Materialstrang (6)
- ein Wollstrang (6-1), oder
- ein Bewässerungsschlauch (6-2), oder
- eine Kombination (6-1, 6-2) daraus ist.

## Claims

1. A weaving loom for inserting continuous material, or loosely connected material or material in pieces, with warp beams (1.a, 1.b) and cloth beam (5), wherein the warp threads (4.a and 4.b) run in the downward direction from the warp beams (1.a and 1.b) to the cloth beam (5) and herein are individually held by hooks (3.a and 3.b) which are anchored in guide beams (2.a and 2.b) between upper warp beams (1.a and 1.b) and lower cloth beam (5), and wherein the warp threads (4.a, 4.b) by way of the disposal of the warp beams (1.a, 1.b) and the disposal and mobility of the guide beams (2.a, 2.b) are movable relative to one another between a V-position which is open at the top and a crossed position.

2. The weaving loom as claimed in claim 1, **characterized in that** the hooks (3.a and 3.b) on the two opposite guide beams (2.a and 2.b) lie opposite one another so as to be mutually offset.

3. The weaving loom as claimed in claim 1 or 2, **characterized in that** at least one of the two guide beams (2.a, 2.b) must be movable so to allow the hooks (3.a and 3.b) to mutually engage when the guide beams (2.a and 2.b) are converged.

4. The weaving loom as claimed in one of claims 1 to 3, **characterized in that** the cloth beam (5) and the guide beams (2.a, 2.b) are disposed and aligned in such a manner that the warp threads (4.a) of the one guide beam (2.a) form a first warp thread plane (17.a), and the warp threads (4.b) of the other guide beam (2.b) form a second warp thread plane (17.b), and that the warp thread planes (17.a, 17.b) by way of a relative movement of the guide beams (2.a, 2.b) having the hooks (3.a, 3.b) are movable relative to one another.

5. The weaving loom as claimed in claim 4, **characterized in that** the warp thread planes (17.a, 17.b) in a relative position form an open V-position, having a shed (18) which is open toward the top, and having the cloth beam (5) below the shed (18).

6. The weaving loom as claimed in claim 5, **characterized in that** the warp thread planes (17.a, 17.b) by means of the guide beams (2.a, 2.b) are movable from the open V-position to a crossed position and back again.

7. The weaving loom as claimed in claim 6, **characterized in that** the warp thread planes (17.a, 17.b) at least in the crossed position can be angled or curved.

8. The weaving loom as claimed in claim 6 or 7, **characterized in that** the warp thread planes (17.a, 17.b) between the V-position and the crossed position can assume a flush position.

9. The weaving loom as claimed in claim 1 or one of the further claims, **characterized in that** at least two warp beams (1.a, 1.b) are provided, each for receiving the warp threads (4.a, 4.b) of one of the warp thread planes (17.a, 17.b), wherein the warp beams (1.a, 1.b) are disposed at a mutual spacing.

10. The weaving loom as claimed in claim 5, **characterized in that** at least two warp beams (1.a, 1.b) are provided, each for receiving the warp threads (4.a, 4.b) of one of the warp thread planes (17.a, 17.b), wherein the warp beams (1.a, 1.b) are disposed at a mutual spacing and the spacing preferably corresponds to at least a largest width of the shed (18) in the V position.

11. The weaving loom as claimed in claim 1 or one of the further claims, **characterized in that** two warp beams (1.a, 1.b) are disposed at a mutual spacing, wherein each of the warp beams (1.a, 1.b) is assigned to one of the guide beams (2.a, 2.b) and holds the warp threads (4.a, 4.b) that are guided by the assigned guide beam (2.a, 2.b).

12. The weaving loom as claimed in claim 11, **characterized in that** the hooks (3.a, 3.b) in the crossed position mutually mesh such that the hooks (3.a) of the one guide beam (2.a) are moved between the hooks (3.b) of the other guide beam (2.b) and past the hooks (3.b) of the latter.

13. The weaving loom as claimed in claim 1 or one of the further claims, **characterized in that** the guide beams (2.a, 2.b) are subdivided into guide beams segments (2.b-1, 2.b-2, 2.b-3, 2.b-4) which are successive in the longitudinal direction, wherein the guide beam segments (2.b-1, 2.b-2, 2.b-3, 2.b-4) are movable in a mutually independent manner.

14. The weaving loom as claimed in claim 1 or one of the further claims, **characterized in that** the hooks (3.a, 3.b) are configured so as to be open, closed, or closed but openable.

15. The weaving loom as claimed in claim 1 or one of the further claims, **characterized in that** the hooks (3.a, 3.b) have an end-proximal hook curvature of at least 280 degrees, preferably at least 370 degrees.

16. The weaving loom as claimed in claim 1 or one of the further claims, **characterized in that** a first frame (9.a) having warp beam (1.a) and guide beam (2.a) is movable relative to a second frame (9.b) having warp beam (1.b) and guide beam (2.b), wherein preferably only one of the frames is movable.

17. A method for producing of woven fabric while using a weaving loom (8) as claimed in one of the preceding claims wherein in the crossed position of the weaving loom (8) a weft thread (7) is introduced into the closed shed (19), and in the V-position of the weaving loom (8) at least one material strand (6) is placed into the then open shed (18), and wherein the material strand differs from the weft thread.

18. The method as claimed in claim 17, **characterized in that** the material strand (6) is
- a wool strand (6-1), or
- a irrigation hose (6-2), or
- a combination (6-1, 6-2) thereof.

## Revendications

1. Métier à tisser pour l'insertion d'un matériau continu, ou à liaison lâche ou en plusieurs parties, avec des ensouples de chaîne (1.a, 1.b) et une ensouple de tissu (5), les fils de chaîne (4.a et 4.b) s'étendant vers le bas depuis les ensouples de chaîne (1.a et 1.b) jusqu'à l'ensouple de tissu (5) et étant ainsi maintenus individuellement par des crochets (3.a et 3.b) qui sont fixés dans des ensouples de guidage (2.a et 2.b) entre les ensouples supérieures (1.a et 1.b) et l'ensouple de tissu inférieure (5), et dans lequel les fils de chaîne (4.a, 4.b) sont aptes à être déplacés les uns par rapport aux autres entre une position en V ouverte vers le haut et une position entrelacée grâce à l'agencement des ensouples de chaîne (1.a, 1.b) et à l'agencement et la mobilité des ensouples de guidage (2.a, 2.b).

2. Métier à tisser selon la revendication 1, **caractérisé en ce que** les crochets (3.a et 3.b) sont décalés les uns par rapport aux autres sur les deux ensouples de guidage (2.a et 2.b) opposées.

3. Métier à tisser selon la revendication 1 ou 2, **caractérisé en ce que**, parmi les deux ensouples de guidage (2.a, 2.b), au moins une doit être mobile pour que, lorsque les ensouples de guidage (2.a et 2.b) se rapprochent l'une de l'autre, les crochets (3.a et 3.b) s'entrelacent entre eux.

4. Métier à tisser selon l'une des revendications 1 à 3, **caractérisé en ce que** l'ensouple de tissu (5) et les ensouples de guidage (2.a, 2.b) sont disposées et orientées de telle sorte que les fils de chaîne (4.a) de l'une des ensouples de guidage (2.a) forment un premier plan (17.a) de fils de chaîne et les fils de chaîne (4.b) de l'autre ensouple de guidage (2.b) forment un deuxième plan (17.b) de fils de chaîne, et **en ce que** les plans de fils de chaîne (17.a, 17.b) sont aptes à être déplacés l'un par rapport à l'autre par un mouvement relatif des ensouples de guidage (2.a, 2.b) avec des crochets (3.a, 3.b) .

5. Métier à tisser selon la revendication 4, **caractérisé en ce que** les plans (17.a, 17.b) des fils de chaîne forment, dans une position relative, une position en V ouverte, avec une foule (18) ouverte vers le haut et avec l'ensouple de tissu (5) en dessous de la foule (18) .

6. Métier à tisser selon la revendication 5, **caractérisé en ce que** les plans (17.a, 17.b) des fils de chaîne sont aptes à être déplacés au moyen des ensouples de guidage (2.a, 2.b) de la position en V ouverte à une position entrelacée, et inversement.

7. Métier à tisser selon la revendication 6, **caractérisé en ce que** les plans (17.a, 17.b) des fils de chaîne sont aptes à être coudés ou courbés au moins dans la position d'entrelacement.

8. Métier à tisser selon la revendication 6 ou 7, **caractérisé en ce que** les plans (17.a, 17.b) de fils de chaîne sont aptes à prendre une position affleurante entre la position en V et la position entrelacée.

9. Métier à tisser selon la revendication 1 ou l'une des autres revendications, **caractérisé en ce qu'**au moins deux ensouples de chaîne (1.a, 1.b) sont prévues, chacune pour recevoir les fils de chaîne (4.a, 4.b) d'un des plans de fils de chaîne (17.a, 17.b), les ensouples de chaîne (1.a, 1.b) étant disposées à distance l'une de l'autre.

10. Métier à tisser selon la revendication 5, **caractérisé en ce qu'**au moins deux ensouples de chaîne (1.a, 1.b) sont prévues, chacune pour recevoir les fils de chaîne (4.a, 4.b) d'un des plans (17.a, 17.b) de fils de chaîne, les ensouples de chaîne (1.a, 1.b) étant agencées à distance l'une de l'autre et la distance correspondant à au moins une largeur maximale de la foule (18) dans la position en V.

11. Métier à tisser selon la revendication 1 ou l'une des autres revendications, **caractérisé en ce que** deux ensouples de chaîne (1.a, 1.b) sont agencées à distance l'une de l'autre, chacune des ensouples de chaîne (1.a, 1.b) étant associée à l'une des ensouples de guidage (2.a, 2.b) et maintenant les fils de chaîne (4.a, 4.b) guidés par l'ensouple de guidage (2.a, 2.b) associée.

12. Métier à tisser selon la revendication 11, **caractérisé en ce que** les crochets (3.a, 3.b) sont imbriqués les uns dans les autres dans la position d'entrelacement de telle sorte que les crochets (3.a) d'une ensouple de guidage (2.a) sont déplacés entre les crochets (3.b) de l'autre ensouple de guidage (2.b) et au-delà de ceux-ci.

13. Métier à tisser selon la revendication 1 ou l'une des autres revendications, **caractérisé en ce que** les ensouples de guidage (2.a, 2.b) sont divisées en segments d'ensouple de guidage (2.b-1, 2.b-2, 2.b-3, 2.b-4) successifs dans le sens de la longueur, les segments d'ensouple de guidage (2.b-1, 2.b-2, 2.b-3, 2.b-4) étant mobiles indépendamment les uns des autres.

14. Métier à tisser selon la revendication 1 ou l'une des autres revendications, **caractérisé en ce que** les crochets (3.a, 3.b) sont conçus de façon à être ouverts, fermés ou fermés mais aptes à être ouverts.

15. Métier à tisser selon la revendication 1 ou l'une des autres revendications, **caractérisé en ce que** les crochets (3.a, 3.b) présentent une courbure d'extrémité de crochet d'au moins 280 degrés, de préférence d'au moins 370 degrés.

16. Métier à tisser selon la revendication 1 ou l'une des autres revendications, **caractérisé en ce qu'**un premier cadre (9.a) avec une ensouple de chaîne (1.a) et une ensouple de guidage (2.a) est mobile par rapport à un deuxième cadre (9.b) avec une ensouple de chaîne (1.b) et une ensouple de guidage (2.b), de préférence un seul des cadres étant mobile.

17. Procédé de fabrication d'un tissu, utilisant un métier à tisser (8) selon l'une des revendications précédentes, dans lequel, dans la position entrelacée du métier à tisser (8), un fil de trame (7) est introduit dans la foule fermée (19), et, dans la position en V du métier à tisser (8), au moins un brin de matériau (6) est placé dans la foule (18) alors ouverte, et dans lequel le brin de matière est différent du fil de trame.

18. Procédé selon la revendication 17, **caractérisé en ce que** le brin de matériau (6) est
- un brin de laine (6-1), ou
- un tuyau d'irrigation (6-2), ou
- ne combinaison (6-1, 6-2) de ceux-ci.
